# EUROPEAN PATENT APPLICATION

(11) **EP 3 939 620 A1**
(43) Date of publication of application: **19.01.2022**
(21) Application number: 20202863.5
(22) Date of filing: 20.10.2020
(51) Int. Cl.: A61L 2/10, F21V 7/00

(54) **SANITIZING CAPSULE**

(30) Priority: 16.07.2020 IT 202000004264 U
(71) Applicant: Slamp S.p.A., 00071 Pomezia(RM) (IT)
(72) Inventor: ZILIANI, Roberto, 00071 Pomezia (RM) (IT)
(74) Representative: Mulas, Chiara

(57) **Abstract**

The present invention relates to a sanitizing capsule, in particular for sanitizing objects from microorganisms such as viruses and bacteria. According to the invention, the sanitizing capsule (10) in particular for objects, is characterized in that it comprises: - a resting plane (1) for resting one or more objects to be sanitized;
- at least one UV rays source (2) at said resting plane (1) adapted to irradiate said one or more objects resting on said resting plane (1) so as to perform a sanitizing cycle;
- a removable cover (3) adapted to be positioned on said resting plane (1) so as to cover said at least one UV rays source (2) and said one or more objects to be sanitized, said removable cover (3) being made by means of the aluminium evaporation metallization technique;
- supply means (4) of said at least one UV rays source (2) for starting said sanitizing cycle;
the coupling between said removable cover (3) and said resting plane (1) being adapted to prevent the propagation of UV rays generated by said at least one UV rays source (2) outside said capsule (10), said sanitizing capsule (10) further comprising a safety system adapted to stop said at least one UV rays source (2) and therefore said sanitizing cycle in case of removal of said cover (3).

## Description

The present invention relates to a sanitizing capsule, in particular for sanitizing objects from microorganisms such as viruses and bacteria.

As is known, the transmission of viruses and bacteria occurs mainly through the respiratory tract, through micro-particles travelling in the air or through aerosols. However, transmission by airways can also occur through indirect contact with contaminated objects, for example through the hands which will then come into contact with the nose and mouth.

In fact, if a person touches the surface of an object or a piece of furniture contaminated by bacteria or viruses with its hands, and then the person rubs its eyes or put its hands on its face without paying attention, there is a high risk of contracting the bacteria or viruses present on that surface.

Therefore, gloves or gels for hand disinfection are used very often to counteract these possible ways of contagion. Frequent hand washing with soap also helps to drastically decrease the risk of contagion.

However, there is another factor that causes contagion through indirect contact: one's personal belongings.

In fact, during the course of the day there are various objects that a person carries around and that without thinking puts in contact with various surfaces in many different places, such as workplaces, means of transport, supermarkets, or that a person touches continuously throughout the day, often without first disinfecting its hands. For example, reference is made to mobile phone, wallet, keys, glasses, etc.

To avoid contagion and in any case to avoid bringing pathogenic organisms into homes through everyday objects one should disinfect them one by one once arrived at home in the evening.

However, this procedure is very slow, and therefore is not always performed. In addition, many objects are complicated to disinfect thoroughly as they have areas where it is difficult to clean.

The object of the present invention is to obviate to the aforementioned drawbacks and in particular to devise a sanitizing capsule for objects which allows the rapid and efficient sanitization of objects.

These and other objects according to the present invention are reached by realizing a sanitizing capsule as explained in claim 1.

Further characteristics of the device are the object of the dependent claims.

The characteristics and advantages of a sanitizing capsule according to the present invention will become more evident from the following, exemplary and nonlimiting description, referred to the attached schematic drawings in which:
- Figure 1 is a front view of an embodiment of a sanitizing capsule according to the invention;
- Figure 2 is a front exploded view of the sanitizing capsule of Figure 1;
- Figure 3 is a perspective exploded view of the sanitizing capsule of Figure 1.

With reference to the figures, a sanitizing capsule is shown, indicated as a whole with 10, in particular for sanitizing objects.

The sanitizing capsule 10 comprises a resting plane 1 for resting one or more objects to be sanitized and at least one UV rays source 2 at the resting plane 1 adapted to irradiate said one or more objects resting on the resting plane 1 so as to perform a sanitizing cycle.

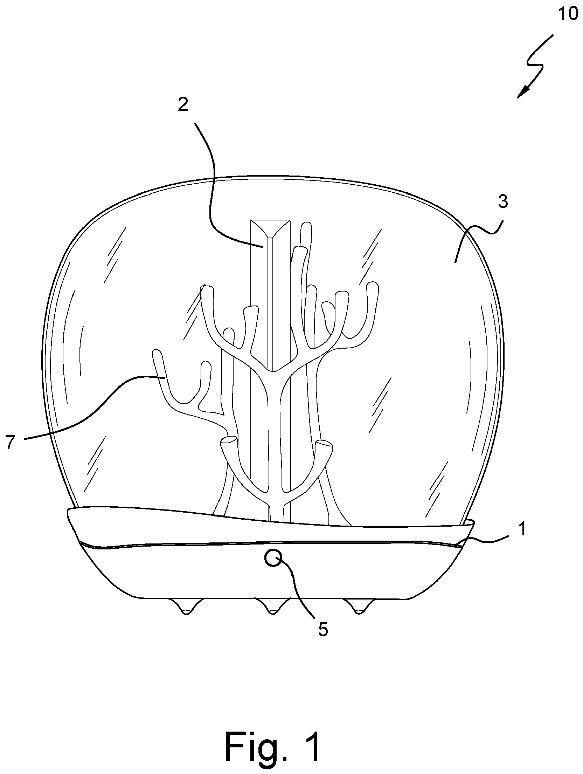

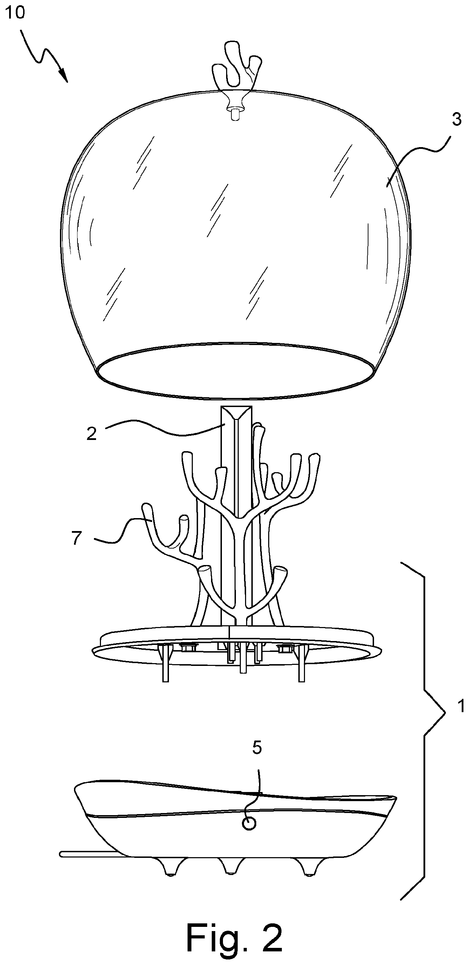

In the embodiment illustrated in Figures 1-3, the capsule 10 comprises a single UV rays source.

The capsule 10 further comprises a removable cover 3 adapted to be positioned on the resting plane 1 so as to cover the UV rays source 2 and said one or more objects to be sanitized.

In particular, the removable cover 3 is made by means of the aluminium evaporation metallization technique which guarantees to obtain a material capable of completely isolating UV rays.

More specifically, the surface treatment of the cover 3 is a deposition metallization which occurs through controlled evaporation of aluminium in an autoclave. The treatment is carried out inside the capsule in order to avoid possible damage during use. The metallization is further protected with a transparent protective varnish, certified for use in an environment saturated with UV rays (A-B-C).

Advantageously, therefore, the coupling between the removable cover 3 and the resting plane 1 is adapted to prevent the propagation of UV rays generated by the at least one UV rays source 2 outside the capsule 10 itself. In fact, the resting plane 1 is also made of a material suitable for blocking the propagation of UV rays.

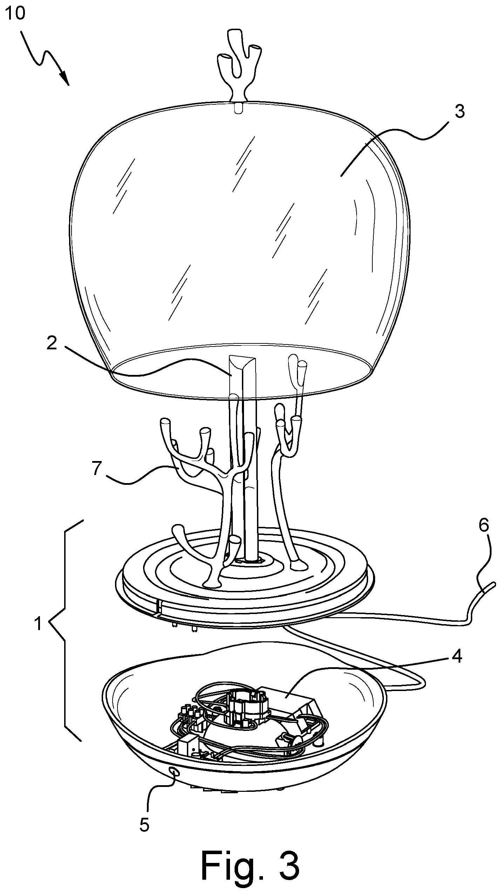

In fact, as can be seen in Figure 3, the resting plane 1 can comprise a shape complementary to the base of the cover 3 so as to ensure a solid closure of the two elements.

Furthermore, the sanitizing capsule 10 comprises a safety system adapted to stop the at least one UV rays source 2 and therefore the sanitizing cycle in case of removal of the cover 3. Said safety system makes it possible to avoid the propagation of UV rays in the external environment in case of accidental removal of the cover 3, which can be highly harmful to the health of the persons who are in the vicinity of the capsule 10.

The capsule 10 comprises supply means 4 for supplying the at least one UV rays source 2 and for starting the sanitizing cycle.

Preferably, the material of the cover 3 also allows it to become transparent when illuminated from the inside by UV rays, which confers particular aesthetic characteristics to the capsule 10.

Preferably, the at least one UV rays source 2 emits UV-C rays having a wavelength comprised in the range between 150 and 350 nm, preferably between 200 and 300 nm, more preferably of about 254 nm.

Advantageously, the sanitizing capsule 10 comprises a power button 5 for commanding the activation of the at least one UV rays source 2 and therefore for starting the sanitizing cycle once the objects to be sanitized have been positioned on the resting plane 1 and have been covered with the cover 3.

Thanks to the safety system, the sanitizing cycle can only be started if the cover 3 has been positioned.

In particular, the safety system provides a mechanical sensor (not illustrated in the attached figures) for checking the correct closure of the cover 3 on the resting plane 1. If the sensor does not detect the correct closure of the cover 3, the power button 5 is not operational.

In an optimal embodiment, the mechanical sensor for checking the positioning of the cover 3 is connected to an electronic control board. The capsule 10 therefore switches on after the electronic control board has detected the position of the cover 3 and subsequently the user has pressed the power button 5. Only then the UV light source 2 switches on to start the sanitizing cycle.

In particular, the sanitizing cycle has a duration comprised between 100 and 300 seconds, preferably between 150 and 250, even more preferably of about 200 seconds.

In the embodiment illustrated in the figures, the sanitizing capsule 10 comprises support elements 7 for the objects to be sanitized placed on the resting plane 1. As can be seen in the figures, the resting plane comprises support elements 7 in the shape of corals on which the objects to be sanitized can advantageously be hung. Said support element 7 allows not only to make the capsule 10 more decorative, but also to ensure a better sanitization of all the surfaces of the objects as they are hung and not resting.

In fact, when an object is resting at least one of its surfaces remains in the shadow.

Furthermore, in the illustrated embodiment, as can be seen in particular in Figure 3, the supply means 4 comprise a power cable 6 adapted to be connected to the electrical network, for example by means of an electric plug. Alternatively, the capsule 10 can comprise a battery which allows it to operate independently. However, in both cases the supply means 4 can be contained in the resting plane 1.

The operation of the sanitizing capsule 10 is as follows.

The capsule 10 is used in particular upon returning to one's home, to sanitize the objects used during the day.

The objects are rested on the resting plane and on the support elements 7 so as to make as many as possible of their surfaces visible to the UV rays source.

Then the removable cover 3 is positioned and the sanitizing cycle is activated by means of the activation button 5, thus the UV rays source is activated.

During this sanitization cycle, which as already mentioned has a duration of about 250 seconds, the UV rays source emits UV rays on the objects that allow to kill all or almost all the bacteria or viruses present on them.

Advantageously, UV-C rays guarantee a 99.99% sanitization and are effective even against the most aggressive strains of bacteria.

If the cover 3 is accidentally removed during the sanitizing cycle, the safety system switches off the UV rays source.

From the above description the features of the device object of the present invention, as well as the advantages thereof, are evident.

Finally, it is clear that the device thus conceived is susceptible to many modifications and variants, all falling within the same inventive concept; furthermore, all details can be replaced by equivalent technical elements. In practice, the materials used, as well as the dimensions thereof, can be of any type according to the technical requirements.

## Claims

1. A sanitizing capsule (10) in particular for objects, **characterized in that** it comprises:
- a resting plane (1) for resting one or more objects to be sanitized;
- at least one UV rays source (2) at said resting plane (1) adapted to irradiate said one or more objects resting on said resting plane (1) so as to perform a sanitizing cycle;
- a removable cover (3) adapted to be positioned on said resting plane (1) so as to cover said at least one UV rays source (2) and said one or more objects to be sanitized, said removable cover (3) being made by means of the aluminium evaporation metallization technique;
- supply means (4) of said at least one UV rays source (2) for starting said sanitizing cycle;
the coupling between said removable cover (3) and said resting plane (1) being adapted to prevent the propagation of UV rays generated by said at least one UV rays source (2) outside said capsule (10),
said sanitizing capsule (10) further comprising a safety system adapted to stop said at least one UV rays source (2) and therefore said sanitizing cycle in case of removal of said cover (3).

2. The sanitizing capsule (10) according to claim 1, wherein said removable cover (3) becomes transparent when illuminated by said UV rays.

3. The sanitizing capsule (10) according to claim 1 or 2, wherein said at least one UV rays source (2) emits UV-C rays having a wavelength comprised in the range between 150 and 350 nm, preferably between 200 and 300 nm, more preferably of about 254 nm.

4. The sanitizing capsule (10) according to one of the preceding claims **characterized in that** it comprises a power button (5) for commanding the activation of said at least one UV rays source (2) and therefore of said sanitizing cycle.

5. The sanitizing capsule (10) according to one of the preceding claims, wherein said sanitizing cycle has a duration comprised between 100 and 300 seconds, preferably between 150 and 250, even more preferably of about 200 seconds.

6. The sanitizing capsule (10) according to one of the preceding claims **characterized in that** it comprises support elements (7) for said objects to be sanitized on said resting plane (1).

7. The sanitizing capsule (10) according to one of the preceding claims, wherein said supply means (4) can comprise a battery inside the capsule or a power cable (6) adapted to be connected to the electrical network.

8. The sanitizing capsule (10) according to one of the preceding claims, wherein said supply means (4) are contained in said resting plane (1).

9. The sanitizing capsule (10) according to one of the preceding claims, wherein said safety system comprises a sensor adapted to detect the correct closure of said cover (3) on said resting plane (1) and an electronic control board in communication with said sensor.
